(19) Europäisches Patentamt
European Patent Office
Office européen des brevets

(11) Publication number: **0 210 828 A2**

(12) **EUROPEAN PATENT APPLICATION**

(21) Application number: **86305637.0**

(22) Date of filing: **22.07.86**

(51) Int. Cl.[4]: **C07D 217/20** , A61K 31/47

(30) Priority: **23.07.85 GB 8518635**

(43) Date of publication of application: **04.02.87 Bulletin 87/06**

(84) Designated Contracting States: **AT BE CH DE FR GB IT LI LU NL SE**

(71) Applicant: **SMITHKLINE BECKMAN CORPORATION**
**One Franklin plaza**
**Philadelphia Pennsylvania 19103(US)**

(72) Inventor: **Kaiser, Carl**
**1105 Sylvan Drive**
**Haddon Heights New Jersey 08035(US)**
Inventor: **Kruse, Lawrence Ivan**
**646 Clinton Avenue**
**Haddonfield New Jersey 08033(US)**

(74) Representative: **Waters, David Martin, Dr. et al**
**Smith Kline & French Laboratories Ltd.**
**Patent Department Mundells**
**Welwyn Garden City Hertfordshire AL7 1EY(GB)**

(54) **Tetrahydroisoquinoline derivatives, process and intermediates for their preparation and pharmaceutical compositions containing them.**

(57) Compounds of structure (I)

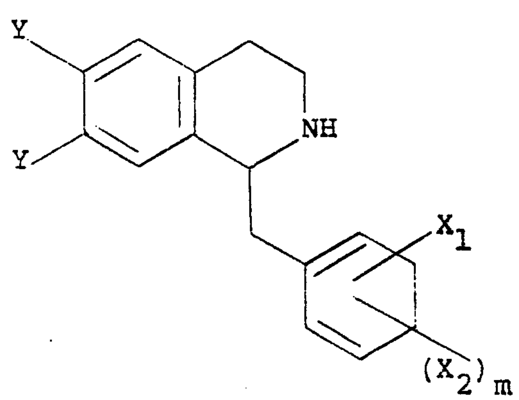

(I)

EP 0 210 828 A2

where Y is OH, $X_1$ is $C_{1-4}$alkoxy, $X_2$ is $C_{1-4}$alkoxy, halogen, $CF_3$ or $C_{1-4}$alkyl, processes for their preparation, compositions containing them and their use as $\beta$-adrenergic receptor antagonists.

## Chemical Compounds

The present invention relates to substituted tetrahydroisoquinoline derivatives which have $\beta$-adrenergic receptor activity, processes for their preparation, intermediates used in their preparation, pharmaceutical compositions containing them, and their use in therapy. Compounds whch antagonize $\beta$-adrenergic receptors ("$\beta$-blockers") are used in the treatment of cardiovascular diseases, for example hypertension, angina pectoris, arrhythmias and obstruction of cardiac outflow. They are also useful in the treatment of glaucoma and migraine headache. Such compounds in common use include, for example, propranolol.

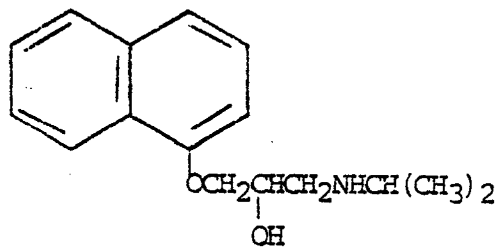

A compound which is known to have the opposite effect that is, to produce a stimulation of the $\beta$-adrenergic receptors and to have bronchodilator activity (Kiyomoto, A., et al., Arzneim.-Forsch. - (Drug Res), 20:46-52, 1970) is trimetoquinol which has the following structure

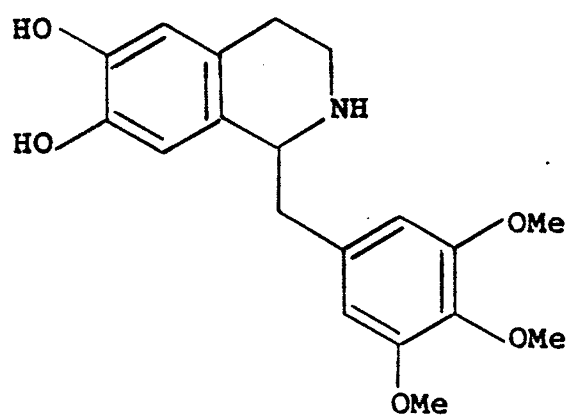

Other dihydroxy tetrahydroisoquinolines are also known. Yamato, et al ., (Tetrahedron, Supp. 8, pt. 1, 129 (1966)) describe compounds of structure

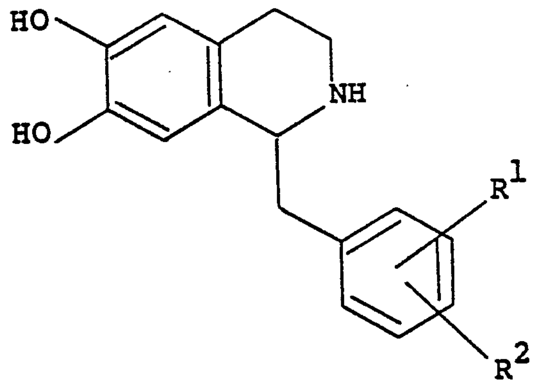

in which $R^1$ and $R^2$ are at the 2, 3 positions and are both hydroxy and their use as anti-depressants and bronchodilating agents. In addition, U.S. Patent No. 3,910,915 describes compounds of the foregoing structure in which $R^1$ and $R^2$ are both halogen or hydroxy, or where one is halogen and the other is hydroxy. No pharmacological utility is disclosed for the compounds.

It has now been found that certain dihydroxy-tetrahydroisoquinolines structurally related to trimetoquinol have significant $\beta$-blocking activity while having little or no $\beta$-receptor stimulating activity and are of use in the treatment of cardiovascular diseases, for example hypertension and other disorders associated with $\beta$-blocking activity.

The present invention therefore provides in a first aspect a compound of structure (I)

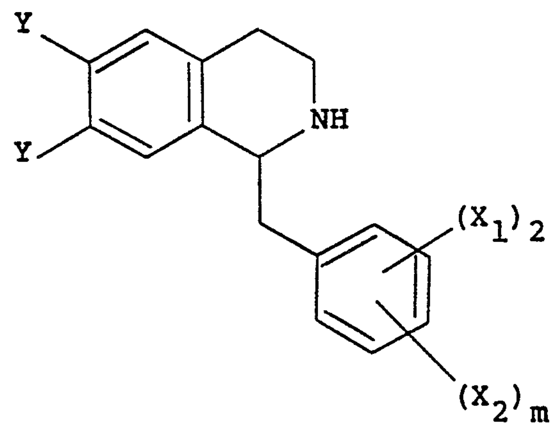

(I)

in which:

Y is hydroxy;

$X_1$ is $C_{1-4}$ alkoxy;

m is 1 to 3; and

$X_2$ is $C_{1-4}$ alkoxy, halogen, $CF_3$ or $C_{1-4}$ alkyl, provided that

i) when m is 1, $X_2$ is halogen, or $C_{1-4}$ alkyl; and,

ii) when m is 2 or 3, $X_2$ is $C_{1-4}$ alkoxy, or a pharmaceutically acceptable acid addition salt thereof.

Suitably, m is 2 or 3. Preferably, m is 1, and $X_2$ is $C_{1-4}$ alkyl, $C_{1-4}$ alkoxy or $CF_3$. Most preferably, m is 1 and $X_2$ is halogen.

$C_{1-4}$ alkyl groups, alone or as part of another group (for example $C_{1-4}$ alkoxy) can be straight or branched, for example methyl, ethyl, n-propyl, i-propyl, i-butyl, s-butyl or n-butyl. Preferably $C_{1-4}$ groups are methyl.

Preferably $C_{1-4}$ alkoxy groups are methoxy or ethoxy.

Preferably halogen groups are chlorine, bromine or iodine.

Particular compounds of the present invention are 6,7-dihydroxy-1-(2,5-dimethoxy-4-bromobenzyl)-1,2,3,4-tetrahydroisoquinoline; 6,7-dihydroxy-1-(2,5-dimethoxy-4-methylbenzyl)-1,2,3,4-tetrahydroisoquinoline; 6,7-dihydroxy-1-(2,3,4,5-tetramethoxybenzyl)-1,2,3,4-tetrahydroisoquinoline; 6,7-dihydroxy-1-(2,3,4,5,6-pentamethoxybenzyl)-1,2,3,4-tetrahydroisoquinoline.

It will be appreciated that the carbon atom of the isoquinoline ring to which the substituted benzyl group is attached is an asymmetric center and thus compounds of formula (I) are optically active compounds. As such the compounds will exist as two optical isomers (enantiomers). Both the pure enantiomers, racemic mixtures (50% of each enantiomer) and unequal mixtures of the two are included within the scope of the present invention.

In a further aspect of the present invention there are provided processes for the preparation of compounds of structure (I). Compounds of structure (I) can be prepared by:

(a) reduction of a compound of structure (II)

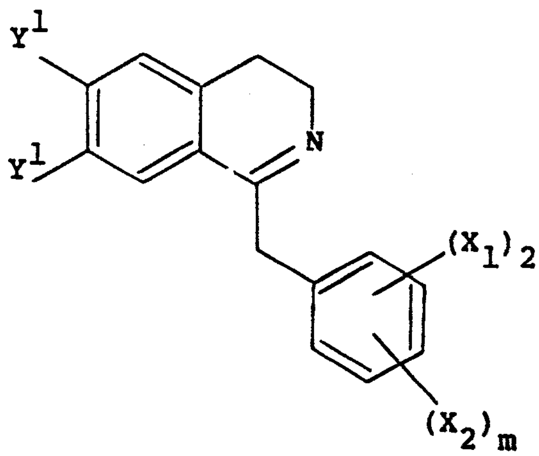

(II)

in which $Y^1$ is a protected hydroxy group, $X_1$, $X_2$ and m are as described for structure (I), to give a compound of structure (IIA)

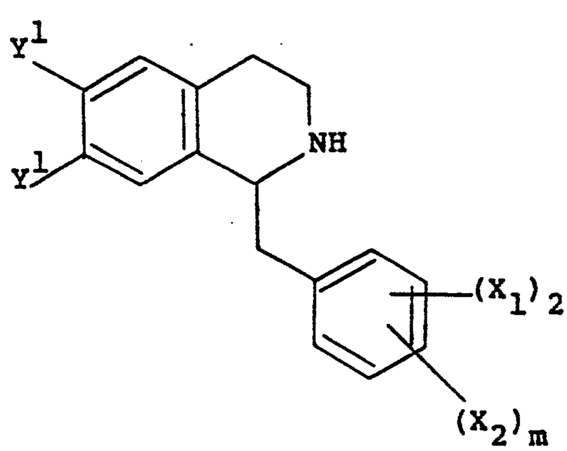

(IIA)

in which $Y^1$, $X_1$, $X_2$ and m are as described for structure (II) followed by deprotection of the protected hydroxy group $Y^1$; or (b) reaction of a compound of structure (III)

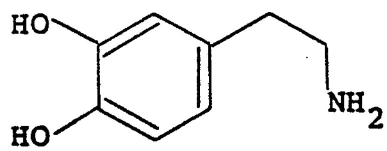

(III)

with a compound of structure (IV)

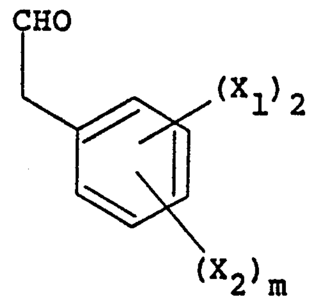

(IV)

in which $X_1$, $X_2$ and m are as described for structure (I); and, optionally, forming a pharmaceutically acceptable salt thereof.

Suitable reducing agents for carrying out step - (a) include for example sodium borohydride in tetrahydrofuran. Suitable protected groups Y' are those groups known in the art which protect the hydroxy group during the reduction step and which can be removed after the reaction is complete. Such groups are described, for example, in "Protective Groups in Organic Synthesis" T.W. Greene, Pub. J. Wiley & Sons, 1981.

Preferably, protected hydroxy groups Y' are benzyl, which can be removed when desired by hydrogenation over a noble metal catalyst, for example, palladium on carbon. The reaction of a compound of structure (III) with a compound of structure (IV) is carried out in a suitable solvent at elevated temperature. Suitable solvents include for example, lower alkanols, preferably ethanol. Preferably the reaction is carried out at the reflux temperature of the solvent used. If it is desired to produce a salt of the compound of structure (I), the corresponding salt of the compound (III) can be used in the reaction with the compound (IV). Suitable salts for such reaction include for example the hydrochloride salt.

Compounds of Structure (II) can be prepared as described in Scheme 1.

Scheme 1

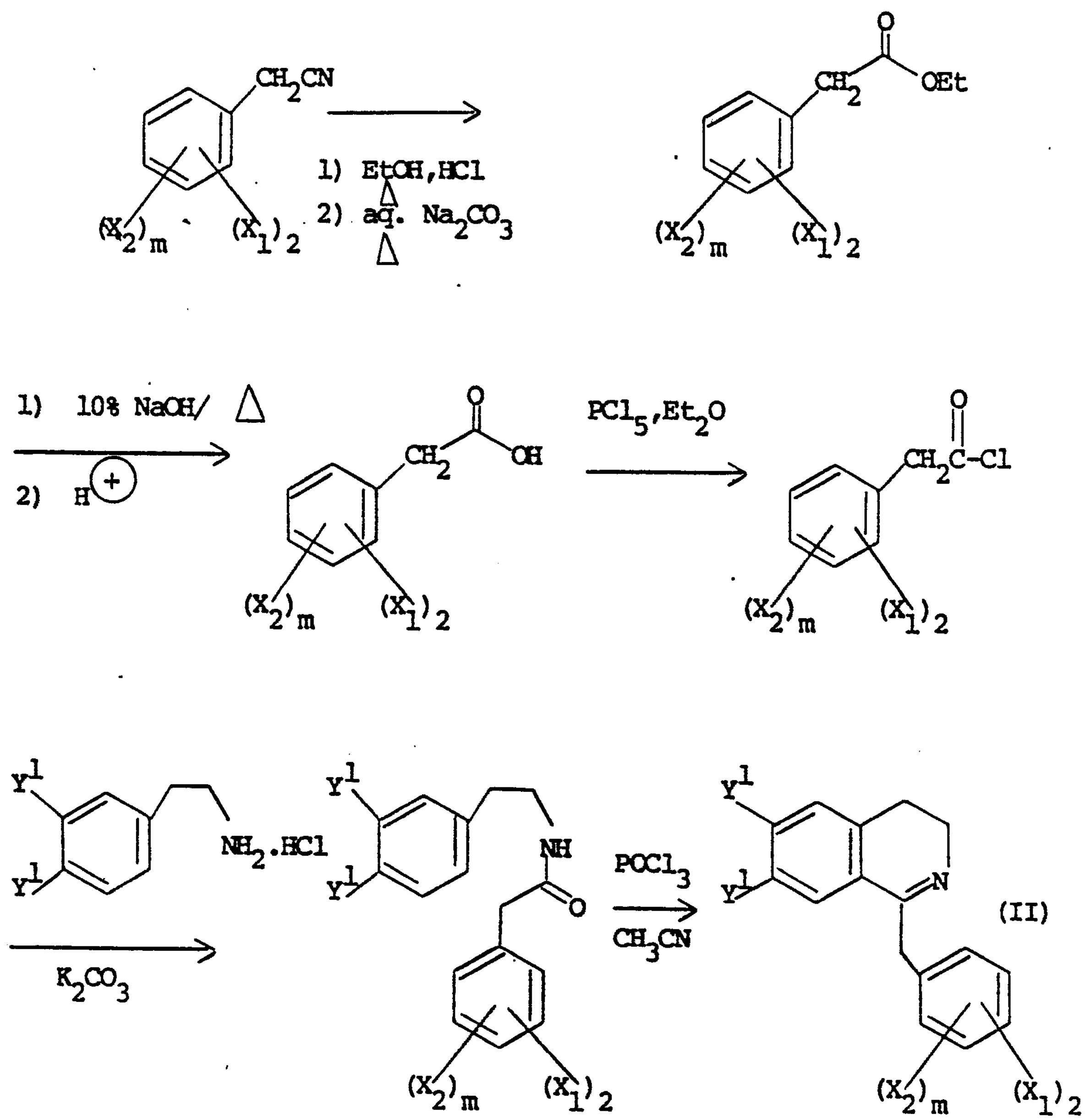


(II)

Compounds of structure (III) and (IV) can be prepared by methods known in the art.

The pharmaceutically acceptable acid addition salts of the compounds of structure (I) are formed with strong or moderately strong organic or inorganic acids by methods known to the art. For example, the base is reacted with an inorganic or organic acid in an aqueous miscible solvent such as ethanol with isolation of the salt by removing the solvent or an an aqueous immiscible solvent when the acid is soluble therein, such as ethyl ether or chloroform, with the desired salt separating directly or being isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

In a further aspect of the present invention there are provided compounds of structure (I) for use as therapeutic agents. The compounds of structure (I) and their pharmaceutically acceptable acid addition salts have been found to antagonize β-receptors. They are of use in the treatment of

cardiovascular diseases, for example hypertension, angina pectoris, arrhythmias and obstruction of cardiac outflow, as well as other disorders, for example, migraine headache and glaucoma.

In therapeutic use, the compounds of the present invention usually are administered in a standard pharmaceutical composition. There is therefore provided in a further aspect of the present invention pharmaceutical compositions comprising a compound of structure (I) or a pharmaceutically acceptable acid addition salt thereof in association with a pharmaceutically acceptable carrier.

The compounds of structure (I) and their pharmaceutically acceptable acid addition salts may be administered in a standard manner for the treatment of the indicated diseases, for example by oral, parenteral, rectal, ocular, or transdermal administration.

For oral or parenteral administration the compounds can be incorporated into convenient dosage unit forms such as capsules, tablets or injectable preparations. Pharmaceutical carriers which can be employed can be solid or liquid. Solid carriers include, among others, lactose, terra alba, sucrose, talc, gelatin, agar, pectin, acacia, magnesium stearate, and stearic acid. Liquid carriers include, among others, syrup, peanut oil, olive oil and water. Similarly, the carrier or diluent may include any time delay material, such as glyceryl monostearate or glyceryl distearate, along or with a wax. The amount of solid carrier will vary widely but, preferably, will be from about 25 mg to about 1 g per dosage unit. If a liquid carrier is used, the preparation will be in the form of a syrup, emulsion, soft gelatin capsule, sterile injectable liquid such as an ampoule, or an aqueous or non-aqueous liquid suspension.

For rectal administration the compositions comprise a binder and/or lubricating agent, for example polymeric glycols, gelatins, cocoa butter or other low-melting vegetable waxes or fats.

For ocular administration the compositions comprise a buffered isotonic liquid, for example isotonic sodium chloride. For transdermal administration, the compositions comprise a conventional aqueous or non-aqueous vehicle, for example a cream, ointment, lotion or paste or can be in the form of a medicated plaster, patch or membrane.

The pharmaceutical preparations are made following conventional techniques of a pharmaceutical chemist involving mixing, granulating and compressing, when necessary, for tablet forms, or mixing, filling and dissolving the ingredients, as appropriate, to give the desired oral or parenteral products.

Doses of the present compounds in a pharmaceutical dosage unit will be an effective amount, that is, a non-toxic quantity selected from the range of 0.1-1000 mg/kg of active compound, preferably 10-100 mg/kg. The selected dose is adminstered to a patient in need of treatment from 1-5 times daily, orally, rectally, by injection or by infusion. Parenteral administration, which uses a low dose, is preferred. However, oral administration, at a higher dose, can also be used when safe and convenient for the patient. For ocular use, sterile isotonic solutions of an acceptable acid addition salt may be administered directly into the eye.

The following examples are illustrative of preparation of compounds of the invention or intermediates thereof. All temperatures and melting points (m.p.) are in degrees Celsius (°C).

EXAMPLE 1

Preparation of 6,7-dihydroxy-1-(2,5-dimethoxy)-4-bromobenzyl)-1,2,3,4-tetrahydroisoquinoline hydrochloride hydrate.

(i) Methyl 3-(2,5,-dimethoxy-4-bromophenyl) glycidate.

To a suspension of 4-bromo-2,5-dimethoxybenzaldehyde (3.9 g, 16 mmole), prepared by bromination of 2,5-dimethoxybenzaldehyde as described by C.F. Barfknecht and D.E. Nichols [ J. Med. Chem., 14 370 1971)], and 1.7 g (16 mmole) of methyl chloroacetate in 35 ml of methanol at 0°C was added dropwise a solution of sodium methoxide in methanol, prepared by cautious addition of 0.4 g (0.017 g-atom) of sodium, in portions, to 25 ml of methanol. The mixture was stirred at 0°C for 30 minutes and then at 25°C for an additional 50 hours. The mixture was poured into an excess of ice-water and acidified with acetic acid to give 4.0 g of a solid which was recrystallized twice from ethyl acetate-cyclohexane to give 1.7 g - (34%) of colorless crystals, m.p. 144-146°C. Analysis calculated for $C_{12}H_{13}BrO_5$: C,45.45; H, 4.13. Found: C,45.47; H, 4.12.

(ii) Sodium 3-(2,5,-dimethoxy-4-bromophenyl)-glycidate.

A solution of sodium methoxide in methanol was prepared by cautious portionwise addition of 0.09 g (0.088 g-atom) sodium to 10 ml of methanol. This solution was added with cooling at 0-5°C to a stirred suspension of 1.2 g (3.8 mmole) of methyl 3-(4-bromo-2,5-dimethoxyphenyl)-glycidate in 10 ml of toluene. After being stirred for 15 minutes,

0.08 ml of water (about 3 drops) was added. Stirring was continued for 3 hours and then ether was added to precipitate a fine white solid that was collected by filtration. After being washed with ether the crystals (1.2 g., 98%) melted 250°C.

(iii) 2,5-Dimethoxy-4-bromophenylacetaldehyde.

A mixture of 0.9 g (2.77 mmole) of sodium 3-(2,5-dimethoxy-4-bromophenyl)glycidate and 0.166 g (2.77 mmole) of acetic acid in 70 ml of toluene was stirred and refluxed under nitrogen for 2.5 hours. The solution was diluted with 50 ml of ether and washed with water. The organic solution was dried ($MgSO_4$) and concentrated to give a 0.4 g of the liquid product that was identified spectroscopically and was shown to be homogeneous by TLC.

(iv) 6,7-Dihydroxy-1-(2,5-dimethoxy-4-bromobenzyl)-1,2,3,4-tetrahydroisoquinoline hydrochloride hydrate

A stirred solution of 0.225 g (1.2 mmole) of 2-(3,4-dihydroxyphenyl)ethylamine hydrochloride and 0.4 g (1.54 mmole) of 2,5-dimethoxy-4-bromophenylacetaldehyde in 50 ml of ethanol was heated under reflux for 3 days. After the solution was concentrated under reduced pressure, the residue was triturated with ethanol to give 0.4 g (63%) of a pink solid, m.p. 153°C, after three recrystallizations from methanol-ether. Analysis calculated for $C_{18}H_{20}BrNO_4$: HCl. 0.5 $H_2O$: C,49.16; H, 5.04; N, 3.19. Found: C, 49.41; H, 5.39; N, 3.11.

EXAMPLE 2

Preparation of 6,7-Dihydroxy-1-(2,5-dimethoxy-4-methylbenzyl)-1,2,3,4-tetrahydroisoquinoline hydrochloride hydrate

(i) Methyl 3-(2,5-dimethoxy-4-methylphenyl) glycidate.

A mixture of 9.0 g (50 mmole) of 2,5-dimethoxy-4-methylbenzaldehyde [R.T. Standridge, et al., J. Med. Chem., 19 1400 (1976)] and 5.5 g - (50 mmole) of methyl chloroacetate in 70 ml of dimethylformamide was added to a stirred suspension of 1.25 g (55 mmole) of sodium hydride in 60 ml of dimethylformamide over a period of 2 hours. The reaction mixture was stirred for 1 more hour, and then it was poured cautiously with stirring into 400 ml of ice-water. The crystalline solid (10 g, 79%) was filtered and recrystallized from ethyl acetatecyclohexane to give colorless crystals, m.p. 101-103°C. Analysis calculated for $C_{13}H_{16}O_5$: C,61.90; H, 6.39. Found: C, 62.26; H, 6.40.

(ii) Sodium 3-(2,5-dimethoxy-4-methylphenylglycidate was obtained as a hygroscopic solid using the same procedure described in Example 1 - (ii).

(iii) 4-Methyl-2,5-dimethoxyphenylacetaldehyde was prepared as a viscous liquid in 41% yield from sodium 3-(2,5-dimethoxy-4-methylphenyl)glycidate in the same manner as described in Example 1 - (iii).

(iv) 6,7-Dihydroxy-1-(2,5-dimethoxy-4-methylbenzyl) 1,2,3,4-tetrahydroisoquinoline hydrochloride hydrate

A mixture of 2.8 g (15 mmole) of 2,5-dimethoxy-4-methylphenylacetaldehyde, 2.0 g - (10.5 mmole) of 2-(3,4-dihydroxyphenyl)ethylamine hydrochloride and 250 ml of ethanol was stirred and refluxed under nitrogen for 3 days. The reaction mixture was evaporated to dryness and washed with ether to give 1.3 g of unreacted 2-(3,4-dihydroxyphenyl)ethylamine hydrochloride. The mother liquors were combined and concentrated to give a semi-solid residue. Trituration of the residue with ethanol followed by recrystallization of the resulting solid from methanol-ether gave 0.4 g of colorless crystals, m.p. 214-216°C. Analysis calculated for $C_{19}H_{23}NO_4$. HCl. 0.25 $H_2O$: C, 61.61; H, 6.67; N. 3.78. Found: C, 61.23; H, 6.66; N, 3.67.

EXAMPLE 3

Preparation 1-(2,3,4,5-tetramethoxybenzyl)-1,2,3,4-tetrahydroisoquinoline hydrochloride hydrate

(i) Methyl 3-(2,3,4,5-Tetramethoxyphenyl)glycidate.

A mixture of 2,3,4,5-tetramethoxybenzaldehyde [L. Syper, K. Kloc and J. Myochowski, Tetrahedron, 36 123 (1980)], methyl chloroacetate in 25 ml dimethylformamide was added dropwise to sodium hydride suspension of 20 ml dimethylformamide keeping reaction temperature around room temperature. After the addition was completed, the mixture was stirred at room temperature for 1 hour (monitored by TLC), poured into ice-water and extracted with ether twice, washed with water three times, brine and dried. The solvent was evaporated

and the residue partitioned between $CH_3CN$ and n-hexane (to remove mineral oil). Evaporation of the $CH_3CN$ layer gave 8.4 g of liquid used for next step (the liquid distilled at 135°C/0.025 mm).

Sodium methoxide solution (15 ml) was added slowly to cooled (5°C) solution of toluene (15 ml) of the product of (ii). After 10 minutes stirring, 0.55 g of water was added. The reaction was stirred for 30 minutes ether added and the reaction stirred again for 2.5h. The resulting pale yellow hygroscopic solid 7.4 g (86%) was collected, washed with ether, and used for next step directly.

(iii) 2,3,4,5-Tetramethoxyphenylacetaldehyde.

A mixture of above sodium salt, 100 ml toluene and 1.5 g glacial acetic acid was refluxed for 2.5 hours under nitrogen, cooled, filtered, and the filtrate washed with water, brine and dried. Evaporation of solvent gave a pale yellow syrup 3 g (52%).

(iv) 1-(2,3,4,5-Tetramethoxybenzyl)-1,2,3,4,-tetrahydroisoquinoline hydrochloride hydrate

A mixture of 1,2,3,4-tetramethoxyphenylacetaldehyde, prepared from step (iii), 2-(3,4-dihydroxyphenyl)ethylamine hydrochloride and 40 ml absolute ethanol was refluxed overnight under nitrogen. The reaction was evaporated and the residue washed with ether. The resulting semi-solid was triturated with ether and recrystallized from methanol/ether to give 1.2 g of solid which contained mostly 2-(3,4-dihydroxyphenyl)ethylamine hydrochloride and some product. The filtrates were combined, evaporated and triturated with minimum amount of ethanol to give 0.2 g of mixture of 2-(3,4-dihydroxyphenyl)ethylamine hydrochloride and product (about 50:50). Mother liquor was again evaporated and triturated with acetonitrile. Solid was pure 2-(3,4-dihydroxyphenyl)ethylamine hydrochloride 2 g. Mother liquor ($CH_3CN$) was evaporated and the residue was recrystallized from methanol/ether twice to give 0.3 g of product, m.p. 130°C (dec.). Analysis: $C_{20}H_{25}NO_6$ HCl. 0.5 $H_2O$. Calculated : C, 57.07; H, 6.47; N, 3.33. Found: C, 56.98; H, 6.70; N, 3.30.

EXAMPLE 4

Preparation of 1-(pentamethoxybenzyl)-6,7-dihydroxy-1,2,3,4-tetrahydroisoquinoline hydrochloride.

(i) Ethyl pentamethoxyphenylacetate.

Pentamethoxyphenylacetonitrile (3.0 g, 0.0112 mole) [F. Berrington, et al., J. Org. Chem., 20, 102 (1953)] was dissolved in 10 ml absolute ethanol and saturated with HCl gas (exothermic) for 15 minutes. The HCl flow was discontinued and the reaction refluxed overnight. Solvent was removed, and then the residue was treated with 20 ml of sodium carbonate solution and heated on a steam bath for 45 minutes. The reaction mixture was diluted with water and extracted with ether, and the ether layer washed with water, brine, dried and evaporated to give 2.7 g of liquid.

(ii) Pentamethoxyphenylacetic acid

The ethyl ester from (i) was mixed with 20 ml 10% NaOH and refluxed for 2 hours. The reaction mixture was extracted with ether, and the aqueous layer made acidic with concentrated HCl and extracted with ether twice.

The ether layer was washed with water, brine and dried ($MgSO_4$). Evaporation of solvent gave solid 2.0 g (62.5 %; based on pentamethoxyphenylacetonitrile). An analytical sample was prepared by recrystallization from cylcohexane/ether, m.p. 100°C.

(iii) Pentamethoxyphenylacetyl chloride

Pentamethoxyphenylacetic acid (0.775 g, 0.0027 g) was dissolved in 15 ml ether and flushed with nitrogen. $PCl_5$ (0.56 g, 0.0027 g) was added portionwise. Stirring was continued for 1 hour - (homogeneous solution), and the mixture evaporated to dryness and used for next step.

(iv) 6,7-Dibenzyloxy-1-(pentamethoxybenzyl)-1,2,3,4-tetrahydroisoquinoline hydrochloride

A solution of pentamethoxyphenylacetyl chloride in 5 ml $CHCl_3$ was added slowly with cooling to a mixture of 2-(3,4-dibenzyloxy)phenethylamine hydrochloride (1.1 g. 0.003 mole), $CHCl_3$ (15 mol), $K_2CO_3$ (1.1 g, 0.08 mole) and water (10 ml). Reaction mixture was stirred at room temperature for 2 hours. The organic layer was separated, washed with water, dilute HCl and with brine, dried, evaporated to give N-[2-(3,4-dibenzyloxyphenylethyl]-2-(pentamethoxyphenyl)acetamide in quantitative yield. The amide was dissolved in 15 ml $CH_3CN$, treated with 0.7 ml $POCl_3$, and refluxed for 2 hours under nitrogen. Evaporation of the mixture gave a residue which was redissolved in $CHCl_3$ and

washed with sodium bicarbonate (5%) solution, brine and dried. Evaporation of solvent gave 6,7-benzyloxy-1-(pentamethoxybenzyl)-3,4-5-dihydroisoquinoline.

The 3,4-dihydroisoquinoline was dissolved in 25 ml methanol and treated with $NaBH_4$ (1.2 g, 0.032 mole) portionwise with cooling and stirring over a 15 minute period, and then stirred at room temperature for 4 hours. Reaction was evaporated to small volume, diluted with water and extracted with ethyl acetate twice. The ethyl acetate layer was washed with water, brine and dried. Removal of solvent gave the HCl salt (0.8 g) 47.6%). An analytical sample was prepared by recrystallization from methanol/ether, m.p. 189-191°C.

(v) 6,7-Dihydroxy-1-(pentamethoxybenzyl)-1,2,3,4-tetrahydroisoquinoline hydrochloride

The product of (iv) (0.8 g, 1.29 mmole) was dissolved in a mixture of 40 ml absolute ethanol and 10 ml methanol and then treated with 0.25 g of 10% Pd/C moistened with 10 ml of ethyl acetate. A few drops of ether/HCl were added, the mixture flushed with nitrogen, and the catalyst removed through glass fibre filter paper. Removal of solvent and recrystallization from methanol/ether gave the required product 0.299 g (48.6%), m.p. 140°C.

EXAMPLES 5-17

Using the procedure substantially as described in Examples 1 to 4, the following compounds are prepared:

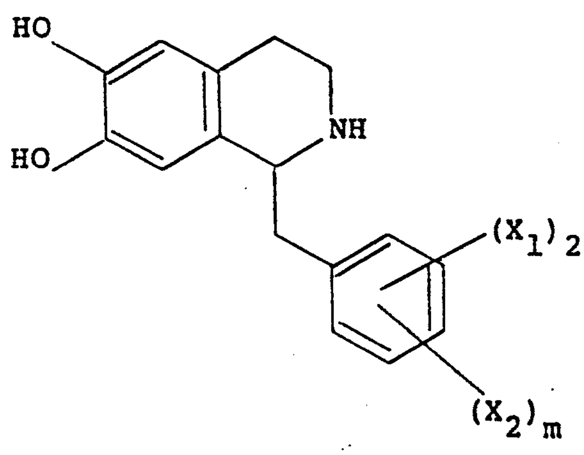

| Example | $X_1$ | $(X_2)_m$ |
|---|---|---|
| 5 | 2,5-$CH_3O$ | 4-Cl |
| 6 | 2,5-$CH_3O$ | 4-$CF_3$ |
| 7 | 2,5-$CH_3O$ | 3-$CH_3O$, 4-Br |
| 8 | 2,5-$CH_3O$ | 3-$CH_3O$, 4-Cl |
| 9 | 2,5-$CH_3O$ | 3-$CH_3O$, 4-$CF_3$ |
| 10 | 2,4-$CH_3O$ | 3-$CH_3O$, 5-Br |
| 11 | 2,4-$CH_3O$ | 3-$CH_3O$,5-Cl |
| 12 | 2,4-$CH_3O$ | 3-$CH_3O$, 5-$CF_3$ |
| 13 | 2,5-$CH_3O$ | 4-$CH_3O$ |
| 14 | 2,5-$CH_3O$ | 4-$CH_3$ |
| 15 | 2,5-$CH_3O$ | 3,4-$CH_3O$ |
| 16 | 2,5-$CH_3O$ | 3,4-$CH_3$ |
| 17 | 2,5-$CH_3O$ | 4-I |

The β-blocking activity of the compounds of structure (I) can be assessed in a test for inhibition of isoproterenol-induced enhancement of the rate of contraction of guinea pig atrial pairs in vitro.

EXAMPLE 18

Inhibition of isoproterenol-induced enhancement of constricted guinea pig atria

A guinea pig (500-700 g) was injected with sodium pentobarbital (50 mg/kg, ip). When anesthetized, the heart was removed and placed in cold (5-10°C), oxygenated Krebs-Hensleit solution. The ventricles and adipose tissue were carefully cut away to leave the atria as an intact pair. A thread was tied to the apex of each atrium and the tissue suspensed in a 50 ml tissue bath, attached to a force-displacement transducer. A diastolic tension of 1g was maintained. Atrial contraction rate was measured using biotachometer, driven by the transducer amplifier.

A 30 minute stabilization period was allowed before obtaining the first dose response curve, during which time the tissue was washed several times with fresh solution. A concentration-response curve to isoproterenol was then determined, increasing agonist concentrations when the rate response to the previous dose had stabilized. After this determination, any β-antagonist activity of the test compound was measured via a concentration-response curve. A concentration of test compound which produced no β-response was then allowed to remain in the tissue bath while the curve for isoproterenol was repeated. The receptor dissociation constant ($K_B$) as a β-antagonist was determined using the formula

$$K_B = \frac{\text{Antagonist Concentration}}{\text{Dose Ratio - 1}}$$

where the Dose Ratio equals the $EC_{50}$ for isoproterenol in the presence of antagonist divided by the control $EC_{50}$ for isoproterenol.

Composition of Krebs-Hensleit Solution = NaCl, 119 mM, $NaHCO_3$, 25 mM; KCl, 4.7 mM, $MgSO_4$, 1.5 mM; $KH_2PO_4$, 1.2 mM; $CaCl_2$, 2.5 mM; glucose, 11 mM; ascorbic acid, $5 \times 10^{-6}$ M; disodium EDTA, $3 \times 10^{-5}$ M. This solution was oxygenated with 95% $O_2$, 5% $CO_2$ and maintained at 37°C.

Results

The results obtained in the above test are given in Table 2.

Table 2

| Compound | $K_B$ on guinea pig atria : (M) |
|---|---|
| Propranolol | $6.2 \times 10^{-10}$ |
| Example 1 | $1.5 \pm 0.9 \times 10^{-8}$ |
| 2 | $6.0 \times 10^{-6}$ |
| 3 | $1.1 \pm 0.6 \times 10^{-6}$ |
| 4 | $1.1 \pm 0.6 \times 10^{-6}$ |

**Claims**

Claims for the Contracting States : BE, CH, DE, FR, GB, IT, LI, LU, NL and SE.

1. A compound of structure (I)

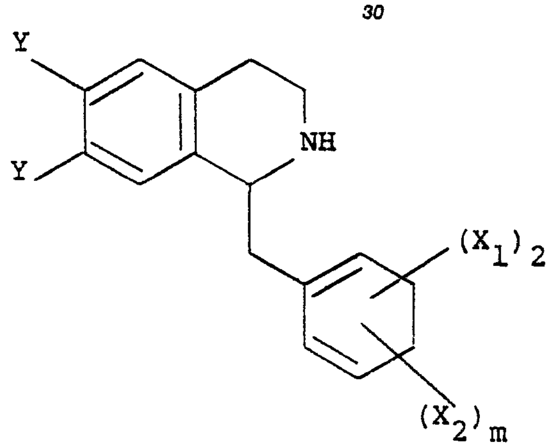

(I)

in which:

Y is hydroxy;

$X_1$ is $C_{1-4}$ alkoxy;

m is 1 to 3; and

$X_2$ is $C_{1-4}$ alkoxy, halogen, $CF_3$ or $C_{1-4}$alkyl, provided that

i) when m is 1, $X_2$ is $CF_3$ halogen, or $C_{1-4}$ alkyl; and,

ii) when m is 2 or 3, at least one $X_2$ is $C_{1-4}$ alkoxy,

or a pharmaceutically acceptable acid addition salt thereof.

2. A compound as claimed in Claim 1 in which m is 1.

3. A compound as claimed in Claim 2 in which $X_2$ is halogen.

4. A compound of structure (I) which is 6,7-dihydroxy-1-(2,5-dimethoxy-4-bromobenzyl)-1,2,3,4-tetrahydroisoquinoline;

6,7-dihydroxy-1-(2,5-dimethoxy-4-methylbenzyl)-

1,2,3,4-tetrahydroisoquinoline;

6,7-dihydroxy-1-(2,3,4,5-tetramethoxybenzyl)-1,2,3,4-tetrahydroisoquinoline; or

6,7-dihydroxy-1-(2,3,4,5,6-pentamethoxybenzyl)-1,2,3,4-tetrahydroisoquinoline.

5. A pharmaceutical composition comprising a compound of Claim 1 in association with a pharmaceutically acceptable carrier.

6. A process for preparing compounds of the following structure (I):

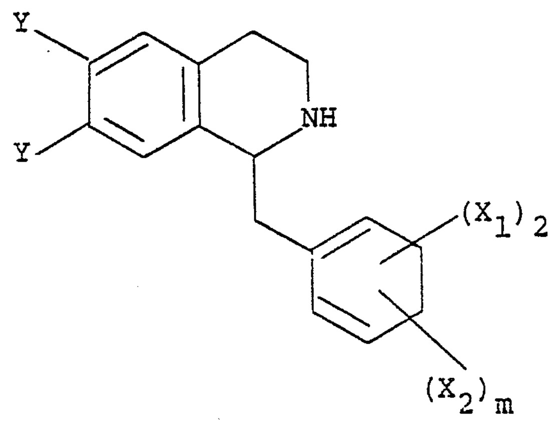

(I)

in which Y, $X_1$, $X_2$, and m are as described in Claim 1 that comprises:

(a) reduction of a compound of structure (II)

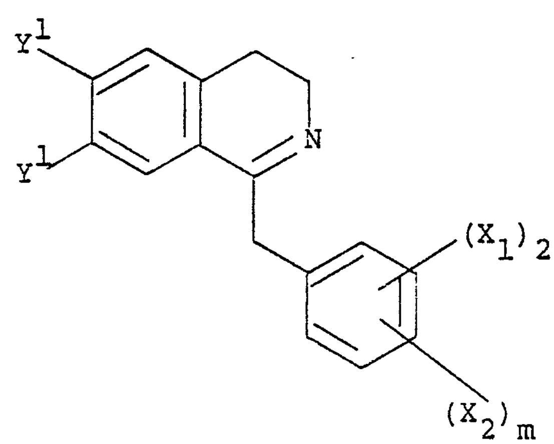

(II)

in which $Y^1$ is a protected hydroxy group and, $X_1$, $X_2$ and m are as described for structure (I), followed by deprotection of the protected hydroxy group $Y^1$; or

(b) reaction of a compound of structure (III)

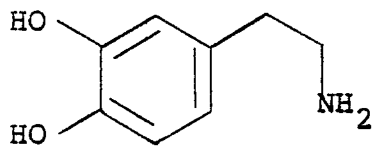

(III)

and a compound of structure (IV)

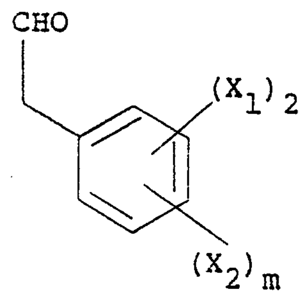

(IV)

in which $X_1$, $X_2$ and m are as described for Claim 1; and optionally, where desired, converting a compound of Claim 1 so formed into a pharmaceutically acceptable acid addition salt thereof.

7. A compound of Claim 1 for use as a therapeutic agent.

8. A compound of Claim 1 for use as a $\beta$-adrenergic receptor antagonist.

9. A compound of structure (II)

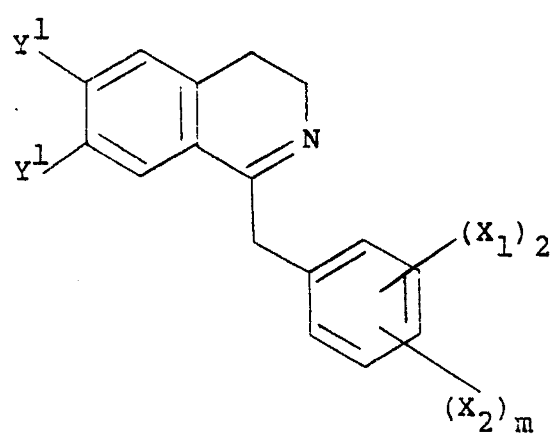

(II)

in which Y' is a protected hydroxy group, and $X_1$, $X_2$ and m are as described in Claim 1.

10. A compound of structure (IIA)

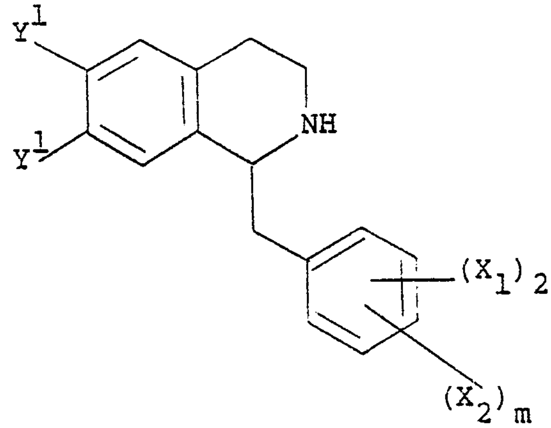

(IIA)

in which $Y^1$, $X_1$ and $X_2$ are as described for structure (II) in Claim 9.

Claims for the Contracting State : AT.

1. A process for the preparation of a compound of structure (I)

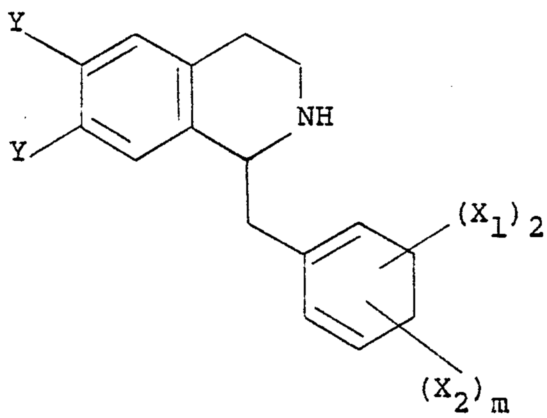

(I)

in which

Y is hydroxy;

$X_1$ is $C_{1-4}$ alkoxy;

m is 1 to 3; and

$X_2$ is $C_{1-4}$alkoxy, halogen, $CF_3$ or $C_{1-4}$alkyl, provided that

i) when m is 1, $X_2$ is $CF_3$, halogen, or $C_{1-4}$ alkyl; and,

ii) when m is 2 or 3, at least one $X_2$ is $C_{1-4}$ alkoxy,

or a pharmaceutically acceptable acid addition salt thereof that comprises.

(a) reduction of a compound of structure (II)

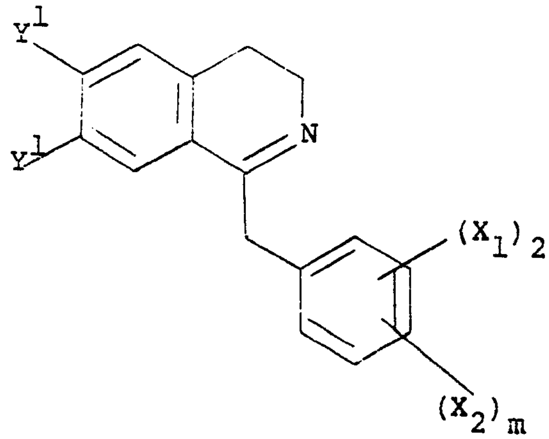

(II)

in which Y' is a protected hydroxy group and, $X_1$, $X_2$ and m are as described for structure (I), followed by deprotection of the protected hydroxy group Y'; or (b) reaction of a compound of structure (III)

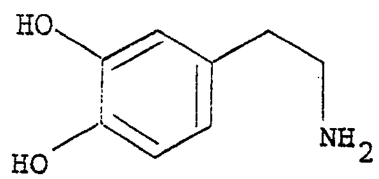

(III)

and a compound of structure (IV)

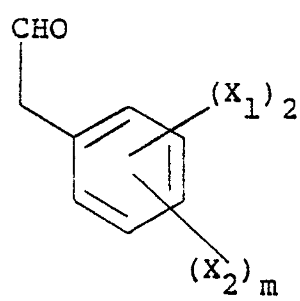

(IV)

in which $X_1$, $X_2$ and m are as described for Claim 1; and optionally, where desired, converting a compound of Claim 1 so formed into a pharmaceutically acceptable acid addition salt thereof.

2. The process of Claim 1 wherein the compound prepared is 6,7-dihydroxy-1-(2,5-dimethoxy-4-bromobenzyl)-1,2,3,4-tetrahydroisoquinoline.

3. The process of Claim 1 wherein the compound prepared is 6,7-dihydroxy-1-(2,3,4,5-tetramethoxybenzyl)-1,2,3,4-tetrahydroisoquinoline.

4. The process of Claim 1 wherein the compound prepared is 6,7-dihydroxy-1-(2,5-dimethoxy-4-methylbenzyl)-1,2,3,4-tetrahydroisoquinoline.

5. The process of Claim 1 wherein the compound prepared is 6,7-dihydroxy-1-(2,3,4,5,6-pentamethoxybenzyl)-1,2,3,4-tetrahydroisoquinoline.

6. A process for the preparation of a pharmaceutical composition which comprises bringing into association a compound of structure (I) or a pharmaceutically acceptable salt thereof and a pharmaceutical carrier.